# EUROPEAN PATENT APPLICATION

(11) **EP 2 745 709 A1**
(43) Date of publication of application: **25.06.2014**
(21) Application number: 12382532.5
(22) Date of filing: 24.12.2012
(51) Int. Cl.: A23L 1/305, A23J 3/10, A23L 1/09, A61K 38/17, C07K 14/47

(54) **Nutritional compositions with reduced beta-casein a1 and related methods**

(71) Applicant: Abbott Laboratories, Inc., Abbott Park, IL 60064 (US)
(72) Inventor: Paul W, Johns, Columbus 43221 (US); Gaurav C, Patel, Gahanna 43230 (US); Rueda Cabrera, Ricardo, 18008 Granada (ES)
(74) Representative: Ungria López, Javier

(57) **Abstract**

Disclosed embodiments provide methods and compositions for improving the metabolic health of consumers. Bovine beta-casein, a major component of milk from cattle, has a variety of genetic variants. Certain genetic variants, A1 in particular, have been associated with type 1 diabetes. The disclosed methods and compositions include reduced amounts of genetic variants of beta-casein that have been associated with diabetes. In some embodiments, the compositions display improved emulsions due to the solubility and hydrophobicity of the genetic variants of beta-casein used therein.

## Description

### TECHNICAL FIELD

Disclosed embodiments are in the field of nutritional compositions and more particularly in the field of nutritional compositions comprising milk proteins having a reduced amount of bovine beta-casein genetic variant A1. Also disclosed are related methods for improving the metabolic health of an individual by consumption of the nutritional compositions.

### BACKGROUND

Proteins sourced from cow's milk are utilized in many nutritional compositions. Much of the milk-sourced proteins used in the United States comes from cows of the breed Holstein-Friesian. Whey and casein make up the vast majority of the protein obtained from cow's milk. While cow's milk from different cattle breeds often has very similar profiles, not all casein proteins contained with the milk are identical. For example, milk proteins from differing cattle breeds may possess different genetic variants of beta-casein. Beta-casein generally has 12 genetic variants: A1, A2, A3, B, C, D, E, F, H1, H2, I, G In particular, the cattle breed Holstein-Friesian has been identified as a breed that produces milk high in beta-casein genetic variant A1. In certain studies, bovine beta-casein genetic variant A1, has been implicated in the onset and development of diabetes and the onset of heart disease. Generally, substances that are found to contribute to the onset of diabetes are termed "diabetogenic." The B, C, F and G genetic variants of beta-casein have also been implicated with the development and onset of diabetes.

### SUMMARY

Provided herein are nutritional compositions and related methods useful for improving the metabolic health of consumers who consume the nutritional compositions. These nutritional compositions comprise protein sourced from cow's milk. Reducing or eliminating the relative amount of bovine beta-casein genetic variant A1 that is contained within a nutritional composition will produce a positive effect on the metabolic health of an individual consuming the nutritional composition. Nutritional compositions that include relatively higher amounts of non-diabetogenic milk proteins A2, A3, D, E, H1, H2, I (and combinations thereof) are expected to improve the metabolic health of individuals who consume them, especially in comparison to consumption of nutritional compositions including diabetogenic variants.

In a first embodiment, a shelf-stable nutritional composition with an improved metabolic profile is provided. The nutritional composition includes at least one protein source in an amount sufficient to provide 5 to 50 % of calories in the nutritional composition from protein, comprising 15 to 100% by weight bovine beta-casein based on the total weight of protein; at least one source of carbohydrates in an amount sufficient to provide 30 to 70 % of calories in the nutritional composition from carbohydrates; and at least one source of fat in an amount sufficient to provide 15 to 60 % of calories in the nutritional composition from fat. The bovine beta-casein comprises at least 80% by weight genetic variants selected from the group consisting of A2, A3, D, E, H1, H2, I, and combinations thereof.

In a second embodiment, a method for improving the metabolic health of an individual is provided. The method comprises: providing a nutritional composition comprising: at least one protein source in an amount sufficient to provide 0.1 to 25 % by weight protein within the nutritional composition (the protein comprising 15 to 100% by weight bovine beta-casein); at least one source of carbohydrates in an amount sufficient to provide 5 to 70 % by weight of the nutritional composition from carbohydrates; and at least one source of fat in an amount sufficient to provide 3 to 60 % by weight of the nutritional composition from fat. The bovine beta-casein comprises less than 50% by weight genetic variants selected from the group consisting of A1, B, C, F and G and combinations thereof; and consumption of the nutritional composition results in improved metabolic health for an individual consuming the nutritional composition.

In a third embodiment, a method for improving the metabolic health of an individual by reducing the *in vivo* production of BCM-7 is provided. The production of BCM-7 is reduced by providing, to the individual, a nutritional composition comprising: at least one source of protein in an amount sufficient to provide 0.1 to 25 % by weight protein within the nutritional composition, the protein comprising 15 to 100% by weight bovine beta-casein; at least one source of carbohydrates in an amount sufficient to provide 5 to 70 % by weight of the nutritional composition from carbohydrates; and at least one source of fat in an amount sufficient to provide 3 to 60 % by weight of the nutritional composition from fat. The bovine beta-casein comprises less than 50% by weight genetic variants selected from the group consisting of A1, B, C, F and G and combinations thereof.

### BRIEF DESCRIPTION OF THE DRAWINGS

FIGURE 1 shows a reversed phase HPLC chromatogram taken at 214 nm of a milk protein isolate available from the New Zealand Milk Products Inc., Santa Rosa, CA, and a milk protein according to the disclosed embodiments having genetic variant A3 as the predominant variant.
FIGURE 2 shows a reversed phase HPLC chromatogram taken at 214 nm of a first milk protein concentrate available from Fonterra USA Inc., Rosemont, IL, and a milk protein according to the disclosed embodiments having genetic variant A3 as the predominant variant.
FIGURE 3 shows a reversed phase HPLC chromatogram taken at 214 nm of a second milk protein concentrate available from Kerry Ingredients & Flavours Beloit, WI, and a milk protein according to the disclosed embodiments having genetic variant A3 as the predominant variant.
FIGURE 4 shows a reversed phase HPLC chromatogram taken at 214 nm of a third milk protein concentrate available from Fonterra, and a milk protein concentrate available from according to the disclosed embodiments having genetic variant A3 as the predominant variant.

### DETAILED DESCRIPTION

Provided herein are nutritional compositions and related methods useful for improving the metabolic health of consumers who consume the nutritional compositions. These nutritional compositions comprise protein sourced from cow's milk. Reducing or eliminating the relative amount of bovine beta-casein genetic variant A1 (and in certain instances B, C, F and G) that is contained within a nutritional composition will produce a positive effect on the metabolic health of an individual consuming the nutritional composition. Nutritional compositions that include relatively higher amounts of non-diabetogenic milk proteins A2, A3, D, E, H1, H2, I (and combinations thereof) are expected to improve the metabolic health of individuals who consume them, especially in comparison to consumption of nutritional compositions including diabetogenic variants.

In a first embodiment, a shelf-stable nutritional composition with an improved metabolic profile is provided. The nutritional composition includes at least one protein source in an amount sufficient to provide 5 to 50 % of calories in the nutritional composition from protein, comprising 15 to 100% by weight bovine beta-casein based on the total weight of protein; at least one source of carbohydrates in an amount sufficient to provide 30 to 70 % of calories in the nutritional composition from carbohydrates; and at least one source of fat in an amount sufficient to provide 15 to 60 % of calories in the nutritional composition from fat. The bovine beta-casein comprises at least 80% by weight genetic variants selected from the group consisting of A2, A3, D, E, H1, H2, I, and combinations thereof.

In a second embodiment, a method for improving the metabolic health of an individual is provided. The method comprises: providing a nutritional composition comprising: at least one protein source in an amount sufficient to provide 0.1 to 25 % by weight protein within the nutritional composition (the protein comprising 15 to 100% by weight bovine beta-casein); at least one source of carbohydrates in an amount sufficient to provide 5 to 70 % by weight of the nutritional composition from carbohydrates; and at least one source of fat in an amount sufficient to provide 3 to 60 % by weight of the nutritional composition from fat. The bovine beta-casein comprises less than 50% by weight genetic variants selected from the group consisting of A1, B, C, F and G and combinations thereof; and consumption of the nutritional composition results in improved metabolic health for an individual consuming the nutritional composition.

In a third embodiment, a method for improving the metabolic health of an individual by reducing the *in vivo* production of BCM-7 is provided. The production of BCM-7 is reduced by providing, to the individual, a nutritional composition comprising: at least one source of protein in an amount sufficient to provide 0.1 to 25 % by weight protein within the nutritional composition, the protein comprising 15 to 100% by weight bovine beta-casein; at least one source of carbohydrates in an amount sufficient to provide 5 to 70 % by weight of the nutritional composition from carbohydrates; and at least one source of fat in an amount sufficient to provide 3 to 60 % by weight of the nutritional composition from fat. The bovine beta-casein comprises less than 50% by weight genetic variants selected from the group consisting of A1, B, C, F and G and combinations thereof.

The discussion of the nutritional compositions herein, should be understood to apply equally to the nutritional compositions useful in the presented methods. Generally, the nutritional compositions and methods described herein are useful for improving the metabolic health of an individual consuming the nutritional composition or following the method. In certain embodiments according to the methods and compositions disclosed herein, the metabolic health of the individual is improved by delaying or preventing the onset of diabetes. In other embodiments according to the methods and compositions disclosed herein, the metabolic health of the individual improved by delaying or preventing the onset of heart disease.

The proteins in bovine milk have been identified as a common source of bioactive peptides. The peptides are released by the digestion of the caseins and whey proteins contained in the milk. In vitro experiments have shown that the bioactive peptide beta-casomorphin 7 (BCM-7) results from successive gastrointestinal proteolytic digestion of bovine beta-casein variants A1 and B. However, the same effect is not seen with variant A2. In experiments performed (*in vitro*) on hydrolyzed milk samples, samples with variant A1 of beta-casein contained BCM-7 levels 4-fold higher than samples with variant A2 milk. *See* Kaminski, S. et al., "Polymorphism of bovine beta-casein and its potential effect on human health," J Appl Genet 48(3), 2007, pp. 189-198. Some research suggests that BCM-7 may play a role in the etiology of human diseases such as heart disease and diabetes (type 1). *Id.* Utilization of the compositions and methods disclosed herein should be useful in reducing the amount of BCM-7 that is generated *in vivo* after the consumption of a nutritional composition containing bovine beta-casein with relatively large amounts of variants A1 and B1 (and variants C, F and G, although these three variants are generally in lower amounts than A1 and B1), and as a result prevent or reduce the onset of diabetes, heart disease or both. It is intended that, consumption of nutritional compositions according to the present disclosure, includes ceasing the consumption of or reduction of consumption of compositions including genetic variants associated with the production of BCM-7.

In certain embodiments according to the first, second and third embodiments disclosed herein, the nutritional compositions are administered (or consumed) orally as needed to provide the desired level of nutrition. In certain of these embodiments, the nutritional compositions are administered (or consumed) in the form of one to two servings daily or in one or two or more divided doses daily. In certain embodiments according to the first and second embodiments, when the nutritional composition is a liquid, the serving may be 150 milliliters to 500 milliliters. In certain other embodiments, when the nutritional composition is a liquid, the serving is 237 milliliters (∼8 fl. oz.). In other embodiments, when the nutritional composition is a liquid, the serving is 177 milliliters to 414 milliliters (∼6 fl. oz. to ∼14 fl. oz.). In yet other embodiments, when the nutritional composition is a liquid, the serving is 207 milliliters to 266 milliliters (∼7 fl. oz. to ∼9 fl. oz.). Various calorie contents may be associated with each serving of the nutritional compositions according to the first, second and third embodiments disclosed herein, typically from 25 to 500 Kcal, including 50 to 400 Kcal; 100 to 350 Kcal or 150 to 350 Kcal per serving. Alternatively, a serving may be construed as any amount which is intended to be consumed in one sitting or within one hour or less.

As previously discussed, in embodiments according to the first, second and third embodiments the protein component of the nutritional compositions includes a specific set of genetic variants of bovine beta-casein. As used herein, the term caseins should be understood to refer to those proteins in bovine milk that will precipitate from the milk at a solution pH of 4.6 (20 °C); caseins typically make up about 80% of the protein in whole, undiluted, bovine milk. Generally, any type of milk-sourced protein source can be utilized for the nutritional compositions according to the first, second and third embodiments as a source of bovine beta-casein, including milk protein concentrate, milk protein isolate, caseinates, and non-fat dry milk, all of which will contain beta-casein.

Bovine beta-casein has further been categorized into several different genetic variants. The genetic variants include A1, A2, A3, B, C, D, E, F, G, H1, H2, and I. Recently, genetic variant A1, B, C, F and G have been associated with an increased incidence of type 1 diabetes. These "diabetogenic" genetic variants are linked in that proteolytic digestion of these variants may result in the release of β-casomorphin-7, associated with several adverse health issues including: heart disease, sudden infant death syndrome and milk allergy. Accordingly, the partial or complete replacement of genetic variants A1, B, C, F and G with non-diabetogenic variants A2, A3, D, E, H1, H2 and I may be expected to confer health benefits to consumers.

The majority of milk protein utilized in milk protein-containing products in the United States is from the cattle breed Holstein. The term "Holstein" as used herein should be understood to encompass the Holstein breed, the Friesian breed and cross-breeds of the two, so-called Holstein-Friesian cattle. Milk from Holstein cattle includes genetic variants A1, B, C, F and G. In contrast, milk from the *Bos indicus* breed does not express A1, B, C, F, or G variants. Similarly, milk from the Guernsey breed of *Bos taurus* has been shown to express very low levels of beta-casein variants A1, B, C or F. Accordingly, embodiments of the first, second and third embodiments include nutritional compositions comprising milk or milk products with very low to no level of the diabetogenic variants, including milk or milk products sourced primarily from Guernsey or *Bos indicus* cattle.

As previously discussed, according to the first embodiment, the nutritional compositions include at least one source of protein in an amount sufficient to provide either 5 to 50 % of the total calories in the shelf-stable nutritional compositions. In embodiments according to the second and third embodiments, the nutritional compositions include at least one source of protein in an amount sufficient to provide 0.1 to 25 % of the total weight of the nutritional composition from protein.

As previously discussed, according to the first, second and third embodiments, the protein component of the nutritional composition comprises 15 to 100% by weight bovine beta-casein (based on the total weight of protein) where that bovine beta-casein comprises at least 80% by weight genetic variants selected from the group consisting of A2, A3, D, E, H1, H2, I and combinations thereof. Thus, as a non-limiting example, in a nutritional composition containing 10 grams of protein per serving, 1.5-10 grams of that protein would be bovine-beta-casein, and at least 1.2-8 grams of the protein would be variants selected from the group consisting of A2, A3, D, E, H1, H2, I and combinations thereof. In certain embodiments according to the first embodiments described herein, the bovine beta-casein comprises at least 90%, at least 95% or even 100% by weight genetic variants selected from the group consisting of A2, A3, D, E, H1, H2, I and combinations thereof. In certain embodiments according to the first, second and third embodiments disclosed herein, the bovine beta-casein comprises 15 to 100% by weight of the total protein, including 15 to 80 % by weight, and including 50 to 100% by weight of the total protein present in the nutritional composition.

In certain embodiments according to the first and second embodiments, the bovine beta-casein used in the nutritional composition comprises at least 80% by weight genetic variants selected from the group consisting of A2, A3, D, E, H1, H2, I, and combinations thereof. In certain embodiments according to the first and second embodiments, the bovine beta-casein comprises at least 80% by weight genetic variants selected from the group consisting of A3, D, E, H1, H2 and I. In certain embodiments according to the first embodiment, the bovine beta-casein comprises at least 90%, at least 95% or even 100% by weight genetic variants selected from the group consisting of A3, D, E, H1, H2 and I. In certain embodiments, the bovine beta-casein comprises less than 10 % by weight (i.e. 0 to 10%, 1 to 10%, 2 to 10%, or 3 to 10%) genetic variant A1, less than 5% or even 0% by weight genetic variant A1. In certain embodiments the predominant (i.e., 50% or more by weight, 50-90% by weight or 50-80% by weight) genetic variant in the bovine beta-casein used in the nutritional composition is genetic variant A3. In embodiments according to the third embodiment, the bovine beta-casein comprises less than 50% by weight genetic variants selected from the group consisting of A1, B, C, F and G and combinations thereof.

The bovine beta-casein utilized in embodiments according to the first, second and third embodiments may be from a single source, or may alternatively be provided by a combination of sources. The bovine beta-casein according to the current disclosure will generally be found in milk protein isolates and milk protein concentrates, but may also be found in other milk protein sources such as non-fat dry milk and caseinates. It may be possible to purify a milk protein isolate (or another milk protein source) containing unacceptably high levels of A1, B, C, F and G genetic variants. Non-limiting examples of purification methods useful for reducing unacceptably high levels of A1, B, C, F and G genetic variants include: a preparative chromatographic process (e.g., affinity chromatography, ion exchange chromatography, reversed phase chromatography) or by a selective salt precipitation (e.g., ammonium sulfate)]. Alternatively, a milk protein source (such as milk protein isolate or milk protein concentrate) containing milk sourced from primarily non-Holstein cattle, and, hence, reduced quantities of A1, B, C, F and G genetic variants may also be utilized in the nutritional compositions and methods disclosed herein. A non-limiting example of a suitable milk protein isolate is CMP-221 Complete Milk Protein Isolate, available from American Casein Company (Burlington, NJ), a powdered milk protein isolate containing 88-89% protein (as is/91-92% dry basis), 1.7-2% fat, 4-5% ash, 1-1.5% carbohydrates, 5-6% moisture and having a pH of 6.6-7.2 when reconstituted in water.

The term milk protein concentrate is generally used to refer to a milk protein containing product that has had a considerable amount of the inherent water from ordinary milk removed and also has had inherent fat from the ordinary milk removed. The term milk protein isolate is generally used to refer to a type of milk-protein containing product that has not only had a considerable amount of the inherent water from ordinary milk removed and inherent fat but also a certain amount of inherent lactose removed. In most instances, milk protein isolates can be considered to be a type of further purified milk protein concentrate. Certain manufacturers may use the term milk protein concentrate to refer to milk-based protein products even if they contain at least 85 weight % protein.

The protein in the nutritional compositions according to the first, second and third embodiments, may be provided by a single source of protein or a combination of protein sources. As previously discussed, 15 to 100% by weight of the protein present in the nutritional composition comprises bovine beta-casein having a particular genetic variant profile. The remaining portion of the protein (i.e., 0-85% by weight of the total protein present in the nutritional composition) can be selected from one or more other sources. As discussed in more detail below, these additional sources of protein are not particularly limited and may include one or more of soy protein, whey protein or any other protein source, including but not limited to those discussed herein. (Furthermore, it should be understood that the source for the remaining portion of the protein could also be selected from milk protein isolate, milk protein concentrate, caseinates, non-fat dry milk that does not meet the previously discussed requirement for having at least 80% by weight genetic variants selected from the group consisting of A2, A3, D, E, H1, H2, I, and combinations thereof as long as these components are not present in amounts so high as to violate other limitations on the amounts of genetic variants discussed previously.)

When soy protein is utilized in the nutritional compositions according to the first, second and third embodiments, the soy protein may be provided by one or more sources of soy protein. Common forms of soy protein include soy protein concentrates, soy protein isolates (including acidified soy protein isolates) and soy protein hydrolysates. As previously discussed, the amount of soy protein utilized in the nutritional compositions is up to 85 weight % of the total protein in certain embodiments (*i.e*., 0-85 weight %) of the total protein, including 0-60 weight %, 0-40 weight %, 0-20, and 0-10 weight % of the total protein, in accordance with the amounts of casein protein discussed above. Commercial sources of soy protein are well known in the nutrition art, non-limiting examples of which include a soy protein isolate distributed by ADM Specialty Products 4666 East Faries Parkway Decatur, IL 62526.

When whey protein is utilized in the nutritional compositions according to the first, second and third embodiments disclosed herein, the whey may be provided by one or more than one source. Common forms of whey protein include whey protein concentrate, whey protein hydrolysate, and whey protein isolate. Various commercial sources of whey protein exist, containing varying concentrations of protein such as about 75 weight % protein (w/w, based on the total weight of the protein source). The amount of whey protein utilized in the nutritional compositions can be up to 85 weight % (i.e., 0-85 weight %) of the total protein, including 0-60 weight %, 0-40 weight %, 0-20, and 0-10 weight % of the total protein in certain embodiments according to the first, second and third embodiments, in accordance with the amounts of casein protein discussed above. Commercial sources of whey protein are well known in the nutrition art, non-limiting examples of which include a whey protein distributed by Hilmar Cheese Company, Hilmar, CA, USA. Milk-sourced whey protein generally contains no caseinates or at most negligible amounts of caseinates, a whey protein source containing a significant component of beta-casein could be utilized in a nutritional composition according to the first, second and third embodiments disclosed herein.

In addition to the protein sources discussed above, in certain embodiments, the nutritional compositions according to the first, second and third embodiments disclosed herein may also contain protein from one or more other sources, in an amount or amounts that is in accordance with the guidance on the amounts of casein proteins noted above. The one or more other sources of protein may in certain embodiments include animal products (*e.g*., meat, fish, egg albumen), cereals (*e.g*., rice, corn), vegetable (*e.g*., soy, pea, potato), or combinations thereof. The additional protein sources can also include, free amino acids known for use in nutritional compositions, non-limiting examples of which include L-tryptophan, L-glutamine, L-tyrosine, L-methionine, L-cysteine, L-arginine" and combinations thereof.

As previously discussed, the nutritional compositions according to the first, embodiment include at least one source of carbohydrates in an amount sufficient to provide 30-70 % of the calories in the nutritional compositions. Further, the nutritional compositions according to the second and third embodiments disclosed herein contain at least one source of carbohydrates in an amount sufficient to provide 5-70% by weight of the nutritional composition The carbohydrates may be provided by one source and, in certain embodiments, more than one source of carbohydrates. The particular amount of carbohydrates present in the nutritional composition may vary depending upon the nutritional needs of the intended user. In certain embodiments, according to the first embodiment the carbohydrates are present in an amount of from 30-50% of the total calories, including from 30 to 45% of the total calories, and alternatively from 35-65% of the total calories in the nutritional compositions; and in certain embodiments according to the second and third embodiments the carbohydrates are present in an amount of from 5-50 % of calories, 10-60 % of calories, or 5-25% of calories.

Various commercial sources of carbohydrates exist and may be utilized in the nutritional compositions disclosed herein. Non-limiting examples of suitable carbohydrates or sources thereof for use in the nutritional compositions disclosed herein include maltodextrin, hydrolyzed or modified starch or cornstarch, glucose polymers, corn syrup, corn syrup solids, rice-derived carbohydrates, glucose, fructose, lactose, high fructose corn syrup, honey, sugar alcohols (*e.g*., malitol, erythritol, sorbitol), slowly digesting carbohydrates, sources of soluble fibers such as resistant starches, gum arabic, pectins, beta-glucans, and the like and combinations thereof.

As previously discussed, the nutritional compositions according to the first embodiment include at least one source of fat in an amount sufficient to provide 15-60% of the calories in the nutritional composition or an amount of 3-60 % by weight of the nutritional composition according to certain embodiments of the second and third embodiments. The fat may be from a single source, or from a variety of sources. The amount of fat present can be characterized as a percentage of total calories in the nutritional composition and may vary depending upon the total number of calories in the nutritional composition. For certain embodiments according to the first embodiment disclosed herein, the amount of fat is within the range of 15 to 60% of the total calories of the nutritional composition, including from 20 to 60% of the total calories, including from 20 to 50% of the total calories and alternatively from 40 to 50% of the total calories in the nutritional compositions. In certain embodiments according to the second and third embodiments disclosed herein, the amount of fat is within the range of 5-60 % by weight, including 5-50 % by weight, 5-25 % by weight, 10-60 % by weight, and 20-60 % by weight of the nutritional compositions.

Various commercial sources of fat exist and may be utilized in the nutritional compositions disclosed herein. Non-limiting examples of suitable fats or sources thereof for use in the nutritional compositions disclosed herein include coconut oil, fractionated coconut oil, soy oil, corn oil, olive oil, safflower oil, high oleic safflower oil, MCT oil (medium chain triglycerides), sunflower oil, high oleic sunflower oil, palm and palm kernel oils, palm olein, canola oil, marine oils, cottonseed oils and combinations thereof.

The nutritional compositions according to the first, second and third embodiments disclosed herein may also optionally include one or more masking agents to reduce or otherwise obscure the development of any residual bitter flavors and after taste in the emulsions over time. Suitable masking agents include natural and artificial sweeteners, sodium sources such as sodium chloride, and hydrocolloids, such as guar gum, xanthan gum, carrageenan, gellan gum, and combinations thereof. The amount of masking agent in the nutritional emulsion may vary depending upon the particular masking agent selected, other ingredients in the formulation, and other formulation or product target variables. Such amounts, however, most typically range from at least 0.1%, including from 0.15% to 3.0%, and also including from 0.18% to 2.5%, by weight of the nutritional composition.

In certain embodiments according to the first, second and third embodiments, consumption of the nutritional compositions results in improved metabolic health for the consumer thereof. The term improved metabolic health is intended to encompass a decrease at least one of the symptoms commonly associated with metabolic syndrome, including: obesity, insulin resistance, hypertension, heart disease, cholesterol abnormalities and other risk factors associated with the onset of diabetes.

In certain embodiments according to the first embodiment, the beta-casein component of the nutritional compositions comprises at least 80% by weight hydrophobic beta-casein variants. One useful measure of hydrophobicity is elution time during reversed phase chromatography. Those of skill in the art will recognize that more hydrophobic entities will elute slower than relatively more hydrophilic moieties. Accordingly, in certain embodiments of the first embodiment, the nutritional compositions comprise beta-casein wherein at least 50 % of the beta-casein in the nutritional composition elutes after genetic variant A1 during reversed phase chromatography.

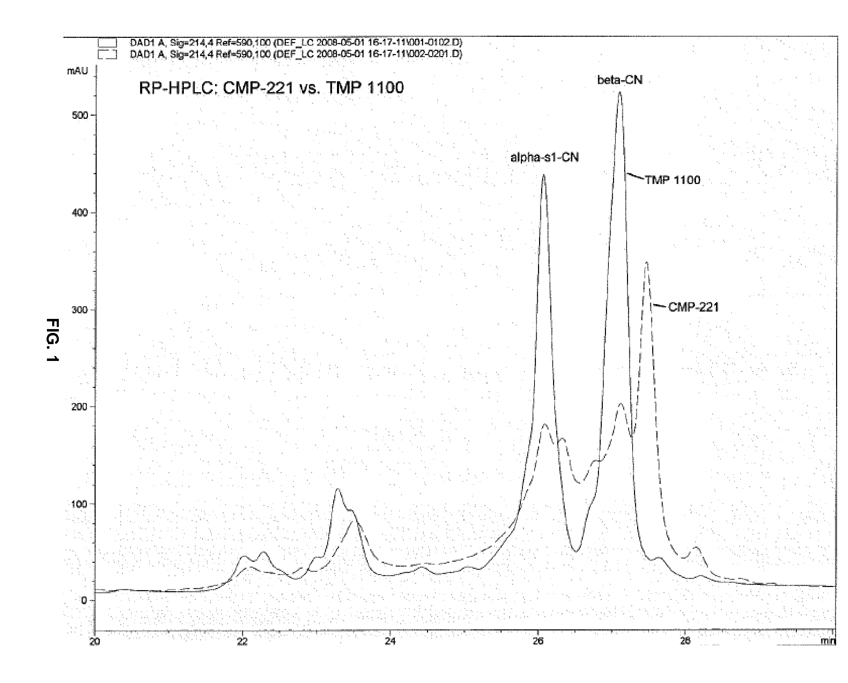

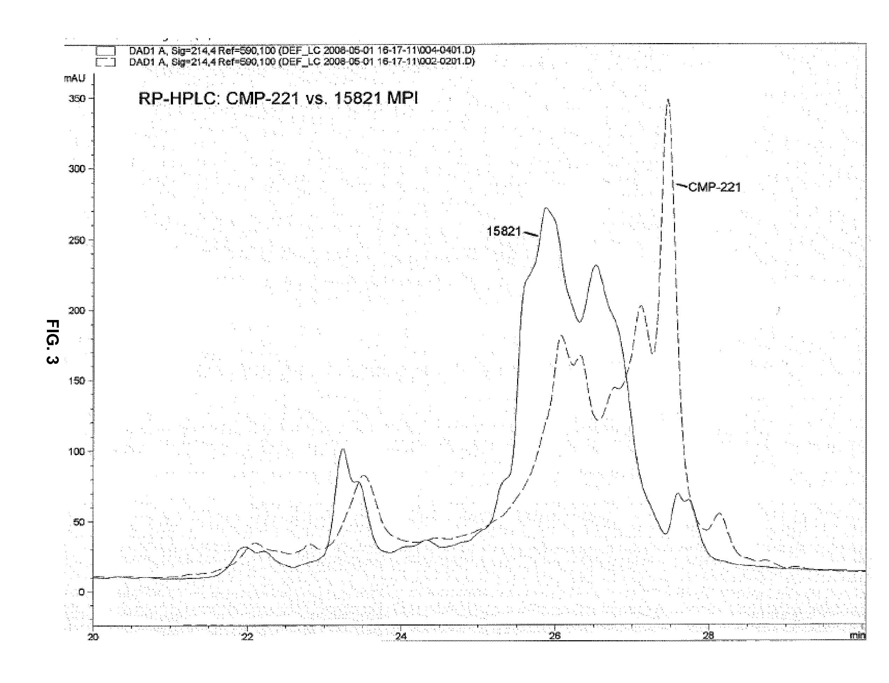

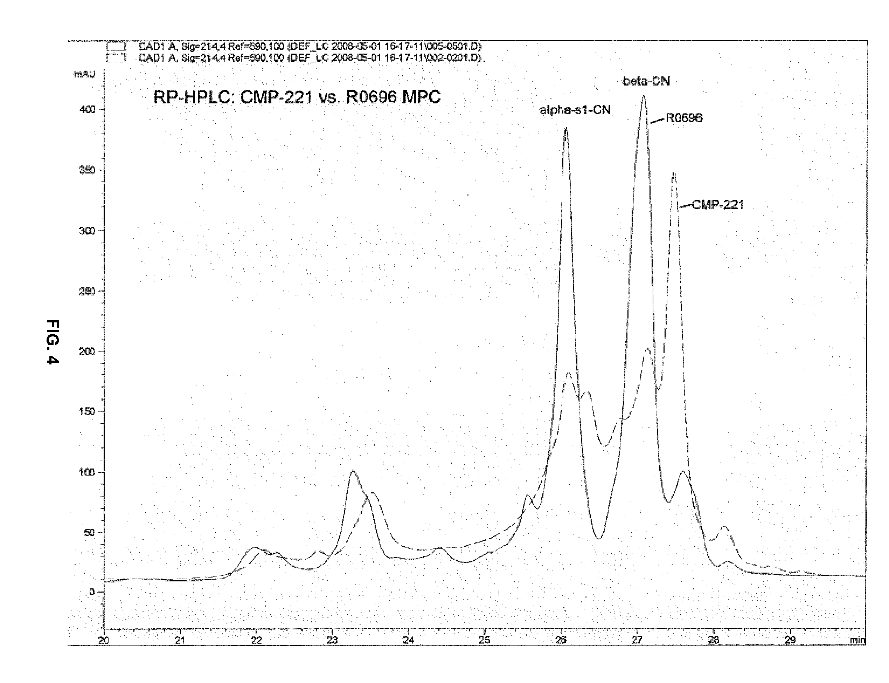

FIGURES 1-4 show side by side HPLC chromatograms comparisons of milk protein sources. Representative conditions for acquisition of the chromatograms include: HPLC SYSTEM: Column: Jupiter C18, 250 x 4.6 mm, 5 um, 300 A, Phenomenex P/N 00G-4053-E0
Eluent A: 800 mL water, 200 mL acetonitrile, 0.5 mL trifluoroacetic acid
Eluent B: 250 mL water, 750 mL acetonitrile, 0.5 mL trifluoroacetic acid
Flow Rate: 0.6 mL/minute
Temperature: 40C
Detection: UV at 214 nm, 280 nm
Injection: 10 uL
Elution Program: 0% B from 0-5 minutes, 0-100% B from 5-40 minutes,
100% B from 40-45 minutes, 0% B from 45-60 minutes (end)

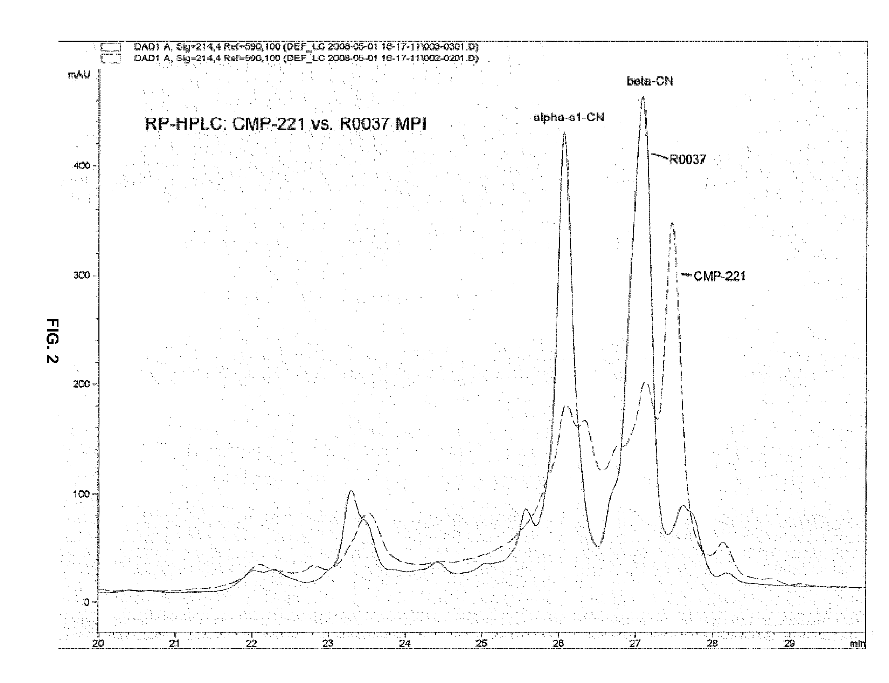

The milk protein concentrates shown in FIGURE 1 include a milk protein isolate having A1 as its predominant genetic variant and a milk protein concentrate (CMP-221) having A3 as its predominant genetic variant. It is clear from the chromatograms that genetic variant A1 elutes faster than other genetic variants and is thus less hydrophobic. Likewise, FIGURES 2-4 show chromatograms of a milk protein concentrate (CMP-221) having A3 as its predominant genetic variant compared to other milk protein concentrates. Although not denoted in the Figures, genetic variant A2 is known to coelute with genetic variant A1 under similar conditions.

The nutritional compositions described herein are useful to provide nutrition, and or to provide individuals one or more benefits as described herein. In accordance with such methods, the nutritional compositions may be administered orally as needed to provide the desired level of nutrition, most typically in the form of one to two servings daily or in one or two or more divided doses daily.

The nutritional compositions described herein (*i.e*., those containing increased amounts of bovine beta-casein variants A2, A3, D, E, H1, H2, I and combinations thereof) should exhibit other advantages as compared to those nutritional compositions containing higher amounts of bovine beta-casein variants A1, B, C, F, G and combinations thereof. For example, a beta-casein source according to the current disclosure (*i.e*., containing increased amounts of bovine beta-casein variants A2, A3, D, E, H1, H2, I and combinations thereof) should exhibit increased solubility, increased hydrophobicity (resulting in better emulsifying capacity) and a decrease in any destabilizing of vitamin B-12 that is present in the nutritional composition. Overall, an increase in emulsifying capacity can be measured through greater physical stability over shelf life of a nutritional composition, and can be evaluated by measuring factors such as separation of the composition and sedimentation (or lack thereof).

The various embodiments of the nutritional composition according to the first, second, and third embodiments disclosed herein may be prepared by any process or suitable method (now known or known in the future) for making a selected product form, such as a nutritional solid, a nutritional powder, or a nutritional liquid. Many such techniques are known for any given product form such as nutritional liquids or nutritional powders and can easily be applied by one of ordinary skill in the art to the various embodiments of the nutritional composition according to the first, second, and third embodiments disclosed herein.

In one suitable manufacturing process for liquid nutritional compositions, for example, at least three separate slurries are prepared, including a protein-in-fat (PIF) slurry, a carbohydrate-mineral (CHO-MIN) slurry, and a protein-in-water (PIW) slurry. The PIF slurry is formed by heating and mixing an oil (*e.g*., canola oil, corn oil, etc.) and then adding an emulsifier (*e.g*., lecithin), fat soluble vitamins, and a portion of the total protein (*e.g*., milk protein concentrate, etc.) with continued heat and agitation. The CHO-MIN slurry is formed by adding with heated agitation to water: minerals (*e.g*., potassium citrate, dipotassium phosphate, sodium citrate, *etc.*), trace and ultra trace minerals (TM/UTM premix), thickening or suspending agents (*e.g*., Avicel® (cellulose), gellan, carrageenan). The resulting CHO-MIN slurry is held for 10 minutes with continued heat and agitation before adding additional minerals (*e.g*., potassium chloride, magnesium carbonate, potassium iodide, *etc*.), and/or carbohydrates (*e.g*., fructooligosaccharide, sucrose, corn syrup, *etc*.). The PIW slurry is then formed by mixing with heat and agitation the remaining protein.

The resulting slurries are then blended together with heated agitation, after which the nutritional composition is subjected to high-temperature short-time (HTST) processing during which the nutritional composition is heat treated, emulsified and homogenized, and then allowed to cool. Water soluble vitamins and ascorbic acid are added, the pH is adjusted to the desired range if necessary, flavors are added, and water is added to achieve the desired total solid level. The nutritional composition is then aseptically packaged to form an aseptically packaged nutritional liquid emulsion. This nutritional liquid emulsion can then be further diluted, heat-treated, and packaged to form a ready-to-feed or concentrated liquid, or it can be heat-treated and subsequently processed and packaged as a reconstitutable powder, *e.g*., spray dried, dry blended, agglomerated.

A nutritional solid, such as a spray dried nutritional powder or dry blended nutritional powder, may be prepared by any collection of known or otherwise effective technique, suitable for making and formulating a nutritional powder.

For example, when the nutritional powder is a spray dried nutritional powder, the spray drying step may likewise include any spray drying technique that is known for or otherwise suitable for use in the production of nutritional powders. Many different spray drying methods and techniques are known for use in the nutrition field, all of which are suitable for use in the manufacture of the spray dried nutritional powders herein.

One method of preparing the spray dried nutritional powder comprises forming and homogenizing an aqueous slurry or liquid comprising predigested fat, and optionally protein, carbohydrate, and other sources of fat, and then spray drying the slurry or liquid to produce a spray dried nutritional powder. The method may further comprise the step of spray drying, dry blending, or otherwise adding additional nutritional ingredients, including any one or more of the ingredients described herein, to the spray dried nutritional powder.

The nutritional compositions disclosed herein can also include other ingredients that may modify the physical, nutritional, chemical, hedonic, or processing characteristics of the product or serve as pharmaceutical or additional nutritional components. Non-limiting examples of such optional ingredients include preservatives, antioxidants, emulsifying agents, buffers, fructooligosaccharides, chromium picolinate, pharmaceutical additives, colorants, flavors or masking agents, thickening agents and stabilizers, artificial sweeteners, hydrocolloids such as guar gum, xanthan gum, carrageenan, gellan gum, gum acacia, and so forth.

The nutritional compositions disclosed herein can also include vitamins, minerals, and combinations thereof. Exemplary vitamins include, but are not limited to, vitamin A, vitamin E, vitamin D2, vitamin D3, vitamin A palmitate, vitamin E acetate, vitamin C palmitate (ascorbyl palmitate), vitamin K, thiamine, riboflavin, pyridoxine, vitamin B12, carotenoids (*e.g*., beta-carotene, zeaxanthin, lutein, lycopene), niacin, folic acid, pantothenic acid, biotin, vitamin C, choline, inositol, salts and derivatives thereof, and combinations thereof. Exemplary minerals include, but are not limited to, calcium, selenium, potassium, iodine, phosphorus, magnesium, iron, zinc, manganese, copper, sodium, molybdenum, chromium, chloride, and combinations thereof.

### EXAMPLES

The following examples illustrate specific and exemplary embodiments and features of the nutritional compositions disclosed herein. The examples are provided solely for the purposes of illustration and should not be construed as limitations of the present disclosure. Numerous variations over these specific examples are possible without departing from the spirit and scope of the presently disclosed nutritional compositions. All amounts indicated within the tables below are weight percentages based upon the total weight of the composition, unless indicated otherwise.

**Example 1:** Table 1 shows partial composition information (protein content) for a commercial liquid nutritional composition compared with an exemplary liquid nutritional composition according to the embodiments disclosed herein. The compositions include a milk protein concentrate (MPC), soy protein concentrate (SPC) combination. The beta-casein utilized in the embodiment on the right includes genetic variant A3 predominantly.

**Table 1**

| **Protein system** | **Current commercial formulation** | **Invention formulation** |
|---|---|---|
| 80% milk protein concentrate (MPC), 20% soy protein concentrate (SPC) | MPC = commodity R0696 | MPC = CMP-221 |
| Total protein concentration | 9 g per 8 oz. serving | 9 g per 8 oz. serving |
| Total β-casein concentration | 1.9 g per 8 oz. serving | 1.9 g per 8 oz. serving |
| Major genetic variant of β-casein | A1 | A3 |

**Example 2:** Table 2 shows partial composition information (protein content) for a commercial liquid nutritional composition compared with an exemplary liquid nutritional composition according to the embodiments disclosed herein. Both illustrate a commercial pediatric liquid nutritional product with varying sources of casein, and have a MPC, whey protein concentrate (WPC), soy protein isolate (SPI) combination. The embodiment on the right has A3 as the major genetic variant.

**Table 2**

| **Protein system** | **Current commercial formulation** | **Invention formulation** |
|---|---|---|
| 65% MPC, 30% whey protein concentrate (WPC), 5% soy protein isolate (SPI) | MPC = commodity R0696 | MPC = CMP-221 |
| Total protein concentration | 7 g per 8 oz. serving | 7 g per 8 oz. serving |
| Total β-casein concentration | 1.2 g per 8 oz. serving | 1.2 g per 8 oz. serving |
| Major genetic variant of β-casein | A1 | A3 |

**Example 3:** Table 3 shows partial composition information (protein content) for a commercial pediatric liquid nutritional composition compared with an exemplary liquid nutritional composition according to the embodiments disclosed herein. Both illustrate liquid nutritional product with varying sources of casein, and have a nonfat dry milk (NFDM), WPC combination. The beta-casein utilized in the embodiment on the right is sourced from Guernsey has A2 as the major genetic variant.

**Table 3**

| **Protein system** | **Current commercial formulation** | **Invention formulation** |
|---|---|---|
| 64% nonfat dry milk (NFDM), 36% WPC | NFDM from Holstein-Friesian breeds | NFDM from Guernsey breed |
| Total protein | 2.07 g per 100 kcal | 2.07 g per 100 kcal |
| concentration | | |
| Total β-casein concentration | 0.36 g per 100 kcal | 0.36 g per 100 kcal |
| Major genetic variant of β-casein | A1 | A2 |

**Example 4:** Table 4 shows partial composition information (protein content) for a commercial liquid nutritional composition designed for individuals with diabetes, compared with an exemplary liquid nutritional composition according to the embodiments disclosed herein. Both illustrate liquid nutritional product with varying sources of caseinates, and have a caseinate, SPC combination. The beta-casein utilized in the embodiment on the right is sourced from *Bos indicus* has A2 as the major genetic variant.

**Table 4**

| **Protein system** | **Current commercial formulation** | **Invention formulation** |
|---|---|---|
| 80% caseinates, 20% SPC | Caseinates from *Bos taurus* milk, Holstein-Friesian breeds | Caseinates from *Bos indicus* milk |
| Total protein concentration | 76 g per liter | 76 g per liter |
| Total β-casein concentration | 22 g per liter | 22 g per liter |
| Major genetic variant of β-casein | A1 | A2 |

**Example 5:** An exemplary liquid nutritional composition suitable for utilizing the protein disclosed herein is described in Table 5 below. The 20 grams of protein comprises 15-100% by weight bovine beta-casein (i.e., 3-20 grams of protein are bovine beta-casein). An exemplary protein source to use to fulfill this requirement would be CMP-221.

**Table 5**

| | **UNIT** | **per 8 fl oz** |
|---|---|---|
| Energy EU | kcal | 350 |
| Protein | g | 20 |
| Fat | g | 11 |
| Linoleic acid | g | 3 |
| Carbohydrate | g | 44 |
| Fructooligosaccharide | g | 3 |
| Sugar | g | 20 |
| Ca-HMB | g | 1.5 |

| VITAMINS | | |
|---|---|---|
| Vitamin A (Palmitate) | IU | 1000 |
| Vitamin A (B-Carotene) | IU | 0 |
| Vitamin D₃ | IU | 160 |
| Vitamin E | IU | 30 |
| Vitamin K₁ | mcg | 20 |
| Vitamin C | mg | 60 |
| Folic Acid | mcg | 200 |
| Vitamin B₁ | mg | 0.38 |
| Vitamin B₂ | mg | 0.43 |
| Vitamin B₆ | mg | 0.5 |
| Vitamin B₁₂ | mcg | 3 |
| Niacin | mg | 5 |
| Pantothenate | mg | 2.5 |
| Biotin | mcg | 75 |
| L-carnitine | mg | 43 |
| Choline | mg | 83 |

| MINERALS | | |
|---|---|---|
| Sodium | mg | 240 |
| Potassium | mg | 560 |
| Chloride | mg | 150 |
| Calcium | mg | 500 |
| Phosphorus | mg | 350 |
| Magnesium | mg | 100 |
| Iron | mg | 4.5 |
| Zinc | mg | 15 |
| Manganese | mg | 0.50 |
| Copper | mg | 0.50 |
| Iodine | mcg | 25 |
| Selenium | mcg | 30 |
| Chromium | mcg | 30 |
| Molybdenum | mcg | 30 |

**Example 6:** An exemplary liquid nutritional composition formulated for use with diabetic subjects and suitable for use with the proteins disclosed herein is provided in Table 6 below. Of the 18% of calories that are provided by protein, 15-100% by weight of that protein is bovine beta-casein, such as can be provided by CMP-221.

**Table 6**

| | UNIT | per 8 fl oz |
|---|---|---|
| **Nutrient Density** | Cal/ml | 0.93 |
| Protein | % Cal | 18 |
| Carbohydrate | % Cal | 47 |
| Fat | % Cal | 35 |
| **Osmolality** | mOsm/ L | 86 |

| **Viscosity** | | Thin |
|---|---|---|
| Protein | g | 9.9 |
| Carbohydrate | g | 29.3 |
| Fat | g | 8.6 |
| Water | g | 200 |
| Ca-HMB | g | 1.5 |
| VITAMINS | | |
| Vitamin A (Palmitate) | IU | 1750 |
| Vitamin A (B-Carotene) | mg | 0.5 |
| Vitamin D | IU | 100 |
| Vitamin E | IU | 30 |
| Vitamin K | mcg | 20 |
| Vitamin C | mg | 60 |
| Folic Acid | mcg | 200 |
| Vitamin B₁ | mg | 0.38 |
| Vitamin B₂ | mg | 0.43 |
| Vitamin B₆ | mg | 1.0 |
| Vitamin B₁₂ | mcg | 3.0 |
| Niacin | mg | 5.0 |
| Pantothenate | mg | 2.5 |
| Biotin | mcg | 75 |
| L-carnitine | mg | 43 |
| Choline | mg | 100 |

| MINERALS | | |
|---|---|---|
| Sodium | mg | 210 |
| Potassium | mg | 370 |
| Chloride | mg | 355 |
| Calcium | mg | 250 |
| Phosphorus | mg | 250 |
| Magnesium | mg | 100 |
| Iron | mg | 4.5 |
| Zinc | mg | 3.8 |
| Manganese | mg | 1.0 |
| Copper | mg | 0.50 |
| Iodine | mcg | 38 |
| Selenium | mcg | 18 |
| Chromium | mcg | 120 |
| Molybdenum | mcg | 38 |

**Example 7:** The table below shows two exemplary liquid nutritional composition formulated for consumption by diabetics and suitable for formulation with the proteins disclosed herein.

**Table 7**

| | | | | **Formula 1** | | **Formula 2** | |
|---|---|---|---|---|---|---|---|
| | | | **kcal/** | **g/L** | **kcal/L** | **g/L** | **kcal/L** |
| **Protein** | | | | | **20%** | | **20%** |
| | Acid Casein | | 4 | 33.90 | 135.6 | 35.00 | 140 |
| | Calcium Caseinate | | 4 | 0.00 | 0 | 5.00 | 20 |
| | SPI | | 4 | 18.00 | 72 | 10.00 | 40 |
| | MPC | | 4 | 3.10 | 32.4 | 0.00 | 0 |
| **Total** | | | 30.00 | 60.00 | 240 | 50.00 | 200 |

| **Fat** | | | | | **45%** | | **49%** |
|---|---|---|---|---|---|---|---|
| | HOSO | | 9 | 28.8 | 262.44 | 27.20 | 244.8 |
| | Marine Oil | | 9 | 2.34 | 21.06 | 0.00 | 0 |
| | Canola | | 9 | 25.86 | 229.5 | 24.48 | 220.32 |
| | Lecithin | | 9 | 3.00 | 27 | 2.72 | 24.48 |
| **Total** | | | | 60 | 540 | 54.4 | 489.6 |

| **CHO** | | | | | **35%** | | **31%** |
|---|---|---|---|---|---|---|---|
| | Maltodextrin | | 4 | 28.3 | 113.2 | 27.2 | 108.8 |
| | Fructose | | 4 | 22 | 88 | 25 | 100 |
| | Maltitol | | 3 | 0 | 0 | 19 | 57 |
| | Sucromalt | | 4 | 3.5 | 38 | 0 | 0 |
| | Glycerol | | 4.3 | 10 | 43 | 0 | 0 |
| | Isomaltulose | | 4 | 24.7 | 98.8 | 0 | 0 |
| | Fibersol (CHO) | | 4 | 3 | 12 | 3.6 | 14.4 |

| | **Fiber** | | | | | | |
|---|---|---|---|---|---|---|---|
| | | Soy Fiber | 1 | 2 | 2 | 12 | 12 |
| | | Oat Fiber | 0.2 | 3 | 0.6 | 0 | 0 |
| | | Fibersol (Fiber) | 2 | 2 | 4 | 2.4 | 4.8 |
| | | FOS | 2 | 10 | 20 | 6.7 | 13.4 |
| **Total** | | | | 114.5 | 419.6 | 95.9 | 310.4 |
| **Total** | | | | | 1200 | | 1000 |

Although certain of the examples presented above include formulations of nutritional compositions that are for ready-to-drink liquid nutritional compositions, other product forms such as concentrated liquids, liquids (*e.g*., suspensions, solutions, emulsions, clear solutions), reconstitutable powders, bars, semi-solids, yogurts, puddings, gels *etc.* may be suitable for the nutritional compositions in various embodiments disclosed herein.

To the extent that the term "includes" or "including" is used in the specification or the claims, it is intended to be inclusive in a manner similar to the term "comprising" as that term is interpreted when employed as a transitional word in a claim. Furthermore, to the extent that the term "or" is employed (*e.g*., A or B) it is intended to mean "A or B or both." When the applicants intend to indicate "only A or B but not both" then the term "only A or B but not both" will be employed. Thus, use of the term "or" herein is the inclusive, and not the exclusive use. See Bryan A. Garner, A Dictionary of Modern Legal Usage 624 (2d. Ed. 1995). Also, to the extent that the terms "in" or "into" are used in the specification or the claims, it is intended to additionally mean "on" or "onto." Furthermore, to the extent the term "connect" is used in the specification or claims, it is intended to mean not only "directly connected to," but also "indirectly connected to" such as connected through another component or components.

While the present application has been illustrated by the description of embodiments thereof, and while the embodiments have been described in considerable detail, it is not the intention of the applicants to restrict or in any way limit the scope of the appended claims to such detail. Additional advantages and modifications will readily appear to those skilled in the art. Therefore, the application, in its broader aspects, is not limited to the specific details, the representative apparatus, and illustrative examples shown and described. Accordingly, departures may be made from such details without departing from the spirit or scope of the applicant's general inventive concept.

## Claims

1. A shelf-stable nutritional composition with an improved metabolic profile comprising:
at least one protein source in an amount sufficient to provide 5 to 50 % of calories in the nutritional composition, the protein comprising 15 to 100% by weight bovine beta-casein based on the total weight of protein;
at least one source of carbohydrates in an amount sufficient to provide 30 to 70 % of calories in the nutritional composition from carbohydrates; and
at least one source of fat in an amount sufficient to provide 15 to 60 % of calories in the nutritional composition from fat,
wherein the bovine beta-casein comprises at least 80% by weight genetic variants selected from the group consisting of A2, A3, D, E, H1, H2, I, and combinations thereof.

2. The nutritional composition of claim 1, wherein the protein comprises 15-80% bovine milk protein.

3. The nutritional composition of claim 1, wherein the bovine beta-casein is sourced from cows other than the breed Holstein.

4. The nutritional composition of claim 1, wherein the bovine beta-casein is sourced from cattle selected from Guernsey and *Bos Indicus* breeds.

5. The nutritional composition of any one claims 1-4, wherein the bovine beta-casein comprises at least 80% by weight genetic variants selected from the group consisting of A3, D, E, H1, H2 and I.

6. The nutritional composition of any one of claims 1-4, wherein the bovine beta-casein comprises less than 10% of genetic variant A1.

7. The nutritional composition of claim 6, wherein the bovine beta-casein is free of genetic variant A1.

8. The nutritional composition of claim 6, wherein the predominant genetic variant within the bovine beta-casein is A3.

9. The nutritional composition of any one of claims 1-4 wherein the liquid nutritional composition was prepared from a reconstitutable powder.

10. The nutritional composition of any of claims 1-4 having 100- 500 kcal per serving wherein a serving is 150-400 mL.

11. The nutritional composition of claim 1 wherein 50 to 100% of the genetic variants of the beta-casein elute after genetic variant A1 if subjected to reverse phase HLPC.

12. The nutritional composition of any of claims 1-4 wherein the composition is a liquid.

13. A method for improving the metabolic health of an individual, comprising:
providing a nutritional composition comprising:
at least one protein source in an amount sufficient to provide 0.1 to 25 % by weight protein within the nutritional composition, the protein comprising 15 to 100% by weight bovine beta-casein;
at least one source of carbohydrates in an amount sufficient to provide 5 to 70 % by weight from carbohydrates in the nutritional composition; and
at least one source of fat in an amount sufficient to provide 3 to 60 % by weight within the nutritional composition from fat,
wherein the bovine beta-casein comprises less than 50% by weight genetic variants selected from the group consisting of A1, B, C, F and G and combinations thereof; and
wherein consumption of the nutritional composition results in improved metabolic health for the individual consuming the nutritional composition.

14. A method according to claim 13, wherein consumption of the nutritional composition results in a decrease in the *in vivo* production of BCM-7.

15. A method according to claim 13 wherein the improved metabolic health comprises preventing or delaying of the onset of diabetes.

16. A method according to claim 13 wherein the improved metabolic health comprises delaying the onset of diabetes.

17. A method according to claim 13 wherein the improved metabolic health comprises preventing or delaying the onset of heart disease.

18. A method according to claim 13 wherein the improved metabolic health comprises delaying the onset of heart disease.

19. The method of any one of claims 13-18 wherein the bovine beta-casein is sourced from cattle other than the breed Holstein.

20. The method of any one of claims 13-18 wherein the bovine beta-casein comprises at least 80% by weight genetic variants selected from the group consisting of A2, A3, D, E, H1, H2, I, and combinations thereof.

21. The method of any one of claims 13-18, wherein the bovine beta-casein is sourced from cattle selected from Guernsey and *Bos Indicus* breeds.

22. The method any one of claims 13-18, wherein the bovine beta-casein comprises at least 80% by weight genetic variants selected from the group consisting of A3, D, E, H1, H2 and I.

23. The method of any one of claims 13-18, wherein the bovine beta-casein comprises less than 10% of genetic variant A1.

24. The method of any one of claims 13-18, wherein the bovine beta-casein is free of genetic variant A1.

25. The method of claim 20, wherein the predominant genetic variant within the bovine beta-casein is A3.

26. The method of any one of claims 13-18 wherein the nutritional composition is a solid nutritional composition.

27. The method of any one of claims 13-18 wherein the nutritional composition is a liquid nutritional composition.

28. The method of claim 27, wherein the liquid nutritional composition is prepared from a reconstitutable powder.

29. The method of any one of claims 13-18 wherein the individual consumes at least two serving per day of the nutritional composition and a serving is 100-400 kcal and 150-400 mL.

30. A method for improving the metabolic health of an individual, comprising reducing the *in vivo* production of BCM-7 by providing a nutritional composition comprising:
at least one source of protein in an amount sufficient to provide 0.1 to 25 % by weight protein within the nutritional composition, the protein comprising 15 to 100% by weight bovine beta-casein;
at least one source of carbohydrates in an amount sufficient to provide 5 to 70 % by weight of the nutritional composition from carbohydrates; and
at least one source of fat in an amount sufficient to provide 3 to 60 % by weight of the nutritional composition from fat,
wherein the bovine beta-casein comprises less than 50% by weight genetic variants selected from the group consisting of A1, B, C, F and G and combinations thereof.
